Europäisches Patentamt

European Patent Office

Office européen des brevets

(19)

(11) **EP 0 676 968 B1**

(12) **EUROPÄISCHE PATENTSCHRIFT**

(45) Veröffentlichungstag und Bekanntmachung des Hinweises auf die Patenterteilung:
**12.05.1999 Patentblatt 1999/19**

(21) Anmeldenummer: **94903812.9**

(22) Anmeldetag: **17.12.1993**

(51) Int. Cl.$^6$: **A61L 15/60**, A61L 15/24

(86) Internationale Anmeldenummer:
**PCT/EP93/03586**

(87) Internationale Veröffentlichungsnummer:
**WO 94/15651 (21.07.1994 Gazette 1994/17)**

(54) **PULVERFÖRMIGE, UNTER BELASTUNG WÄSSRIGE FLÜSSIGKEITEN SOWIE BLUT ABSORBIERENDE POLYMERE, VERFAHREN ZU IHRER HERSTELLUNG UND IHRE VERWENDUNG IN TEXTILEN KONSTRUKTIONEN FÜR DIE KÖRPERHYGIENE**

POWDER-FORM POLYMERS WHICH ABSORB, EVEN UNDER PRESSURE, AQUEOUS LIQUIDS AND BLOOD, A METHOD OF PRODUCING THEM AND THEIR USE IN TEXTILE ARTICLES FOR BODY-HYGIENE APPLICATIONS

POLYMERES PULVERULENTS ABSORBANT EN CHARGE LES LIQUIDES AQUEUX ET LE SANG, PROCEDE POUR LEUR PREPARATION ET UTILISATION DANS DES PRODUITS TEXTILES POUR L'HYGIENE CORPORELLE

(84) Benannte Vertragsstaaten:
**AT BE CH DE DK ES FR GB GR IE IT LI LU MC NL PT SE**

(30) Priorität: **30.12.1992 DE 4244548**

(43) Veröffentlichungstag der Anmeldung:
**18.10.1995 Patentblatt 1995/42**

(73) Patentinhaber:
**Stockhausen GmbH & Co. KG
47805 Krefeld (DE)**

(72) Erfinder:
• **KLIMMEK, Helmut
D-47803 Krefeld (DE)**
• **BREHM, Helmut
D-47800 Krefeld (DE)**

(74) Vertreter: **Wolff, Felix et al
Kutzenberger & Wolff
Theodor-Heuss-Ring 23
50668 Köln (DE)**

(56) Entgegenhaltungen:
EP-A- 0 415 183     EP-A- 0 450 924
WO-A-91/15362     DE-C- 4 020 780
US-E- R E32 649

**Beschreibung**

Die Erfindung betrifft pulverförmige, vernetzte, wäßrige Flüssigkeiten sowie Blut absorbierende Polymeren (Superabsorber) mit verbesserten Eigenschaften hinsichtlich Quellung und Rückhaltevermögen von wäßrigen Flüssigkeiten unter Belastung, ein Verfahren zur Herstellung dieser Polymeren sowie ihre Verwendung in absorbierenden Sanitärartikeln wie Babywindeln, bei der Erwachseneninkontinenz, der Damenhygiene und der Wundabdeckung.

Superabsorber sind wasserunlösliche, vernetzte Polymeren, die in der Lage sind, unter Quellung und Ausbildung von Hydrogelen große Mengen an wäßrigen Flüssigkeiten und Körperflüssigkeiten, wie Urin oder Blut, aufzunehmen und die absorbierte Flüssigkeitsmenge unter einem bestimmten Druck zurückzuhalten. Durch diese charakteristischen Absorptionseigenschaften finden die Polymeren hauptsächlich Anwendung bei der Einarbeitung in Sanitärartikel, z. B. in Babywindeln und Damenbinden.

Bei den gegenwärtig kommerziell verfügbaren Superabsorbern handelt es sich um vernetzte Polyacrylsäuren oder vernetzte Stärke-Acrylsäure-Pfropfpolymerisate, bei denen die Carboxylgruppen teilweise mit Natronlauge oder Kalilauge neutralisiert sind. Die Herstellung der pulverförmigen Superabsorber erfolgt prinzipell nach bei Methoden:

Nach der ersten Methode wird teilneutralisierte Acrylsäure in wäßriger Lösung in Gegenwart eines mehrfunktionellen Vernetzers durch radikalische Polymerisation in ein Gel überführt, das dann zerkleinert, getrocknet, gemahlen und auf die gewünschte Partikelgröße abgesiebt wird. Diese Lösungspolymerisation kann kontinuierlich oder diskontinuierlich durchgeführt werden. Typische Verfahren sind z. B. beschrieben in US PS 42 86 082, DE PS 27 06 135 und US PS 40 76 663.

Die zweite Methode ist das inverse Suspensions- und Emulsionspolymerisationsverfahren. In diesem Prozeß wird eine wäßrige, teilneutralisierte Acrylsäurelösung mit Hilfe von Schutzkolloiden oder Emulgatoren in einem hydrophoben, organischen Lösungsmittel dispergiert und durch Radikalinitiatoren die Polymerisation gestartet. Nach Beendigung der Polymerisation wird das Wasser aus dem Reaktionsgemisch azeotrop entfernt und das Polymerprodukt abfiltriert und getrocknet. Die Vernetzungsreaktion kann durch Einpolymerisieren eines polyfunktionellen Vernetzers, der in der Monomerenlösung gelöst ist, und/oder durch Reaktion geeigneter Vernetzungsmittel mit funktionellen Gruppen des Polymeren während einer der Herstellungsschritte erfolgen. Das Verfahrensprinzip ist z. B. in US PS 43 40 706, DE PS 37 13 601 und DE PS 28 40 010 beschrieben.

Während in der Entwicklung der Superabsorber zunächst allein das sehr hohe Quellvermögen bei Kontakt mit Flüssigkeit, auch freie Quellkapazität genannt, im Vordergrund stand, hat sich später gezeigt, daß es nicht nur auf die Menge der absorbierten Flüssigkeit ankommt, sondern auch auf die Festigkeit des gequollenen Gels. Absorptionsvermögen, Quellvermögen oder freie Quellkapazität genannt einerseits, und Gelfestigkeit bei einem vernetzten Polymeren andererseits, stellen jedoch gegenläufige Eigenschaften dar, wie bereits aus der US PS 32 47 171 und ferner aus der US PS Re 32 649 bekannt ist. Das bedeutet, daß Polymeren mit besonders hohem Absorptionsvermögen nur eine geringe Festigkeit des gequollenen Gels aufweisen mit der Folge, daß das Gel unter einem angewendeten Druck (z. B. Körperdruck) deformierbar ist und die weitere Flüssigkeitsverteilung und Flüssigkeitsaufnahme verhindert. Nach der US PS Re 32 649 soll daher ein ausgewogenes Verhältnis derartiger Superabsorber in einer Windelkonstruktion Flüssigkeitsaufnahme, Flüssigkeitstransport, Trockenheit der Windel und der Haut gewährleisten. Dabei kommt es nicht nur darauf an, daß das Polymere Flüssigkeit unter nachfolgender Einwirkung eines Drucks zurückhalten kann, nachdem das Polymere zunächst frei quellen konnte, sondern auch darauf, Flüssigkeiten auch gegen einen gleichzeitig, d. h. während der Flüssigkeitsabsorption, ausgeübten Druck aufzunehmen, wie es unter praktischen Gegebenheiten geschieht, wenn ein Baby oder eine Person aufeinem Sanitärartikel sitzt oder liegt, oder wenn es beispielsweise durch Beinbewegung zur Einwirkung von Scherkräften kommt. Diese spezifische Absorptionseigenschaft wird in der EP 03 39 461 A1 als Aufnahme unter Druck bezeichnet.

Der zunehmenden Tendenz, aus verständlichen ästhetischen Gründen und aus Umweltaspekten (Verringerung des Deponievolumens), die Sanitärartikel immer kleiner und dünner zu gestalten, kann nur dadurch entsprochen werden, daß man den großvolumigen Fluffanteil in Windeln reduziert und gleichzeitig den Anteil an Superabsorber erhöht. Hierdurch muß der Superabsorber zusätzlich Aufgaben bezüglich Flüssigkeitsaufnahme und -transport übernehmen, die vorher der Fluff erfüllte.

Wird der Anteil des Superabsorbers im Hygieneartikel, z. B. in einer Windel, auf 40 % oder gar auf 60 % und darüber hinaus erhöht, werden kommerziell erhältliche Superabsorber praktisch unbrauchbar. Die Flüssigkeitsaufnahme, besonders unter Druck, wird viel zu langsam. Die Teilchen neigen dazu, ein 'koaguliertes Gel' zu bilden. Die so entstandene Gelbarriere blockiert den weiteren Flüssigkeitstransport. Dieses Phänomen wird bekanntlich als 'gel blokking' bezeichnet.

Um superabsorbierende Polymeren bereitzustellen, die die besondere Eigenschaftskombination, wie hohe Retentionskapazität, hohe Gelstärke und hohes Aufnahmevermögen unter Druck besitzen, ist es erforderlich, die pulverförmigen Polymeren nachträglich zu behandeln.

Nach der GB 21 19 384 A wird durch die Behandlung der Polymeren mit Verbindungen, die mindestens zwei funktionelle Gruppen tragen, die mit den Carboxylgruppen des teilchenförmigen Polymeren in der Oberflächenschicht

2

reagieren können, eine deutliche Eigenschaftsverbesserung erreicht.

In der DE 0S 35 23 617 wird ein Verfahren zur Nachbehandlung von pulverförmigen Polymeren mit einem mehrwertigen Alkohol beschrieben, der unverdünnt, verdünnt mit Wasser und/oder organischem Lösungsmittel vor der Reaktion bei erhöhter Temperatur auf das pulverförmige Polymere aufgetragen wird.

Nach DE-PS 40 20 780 wird die verbesserte Quellfähigkeit eines superabsorbierenden Polymeren gegen Druck durch die Erwärmung des Polymerpulvers mit 0,1 - 5 Gew.-% Alkylencarbonat, das gegebenenfalls mit Wasser und/oder Alkohol verdünnt aufgetragen wurde, erreicht.

Wie in DE 0S 35 23 617 wird auch in EP 04 50 924 A2 die Behandlung der Oberfläche eines absorbierenden Polymeren mit einem Polyol, das gegebenenfalls mit Wasser und/oder organischem Lösungsmittel verdünnt eingesetzt wird, durchgeführt. In dieser Veröffentlichung wird ausführlich auf die Bedeutung der Verdünnung des Behandlungsmittels mit Wasser und/oder organischem Lösungsmittel eingegangen. Bei einer Verdünnung des zur Reaktion mit den Carboxylgruppen des Polymeren befähigten Behandlungsmittels ausschließlich mit Wasser ergeben sich enorme verfahrenstechnische Schwierigkeiten. Das pulverförmige, wasserquellbare Polymere verbackt bei Kontakt mit Wasser oder wäßrigen Lösungen, so daß eine homogene Verteilung des Behandlungsmittels auf die Partikeloberfläche unmöglich ist. Will man auf Wasser bei der Vermischung von wasserquellbarem, pulverförmigem Polymeren mit einer Verbindung, die mit den Carboxylgruppen des Polymeren reagieren kann, nicht verzichten, um den Diffusionsprozeß der Behandlungsmittel in den Feststoff zu unterstützen, so ist man gezwungen, das Wasser durch einen Überschuß Behandlungsmittel oder ein nicht reaktives organisches Lösungsmittel zu inertisieren. Inertisieren mit einem organischen Lösungsmittel bedeutet die Verwendung von Flüssigkeiten, die das Polymer nicht quellen, also beim Mischvorgang mit dem pulverförmigen Polymeren nicht zu Verbackungen führt.

Dem Fachmann ist die Schwierigkeit bekannt, geringe Flüssigkeitsmengen mit pulverförmigen Substanzen homogen zu vermischen, insbesondere wenn eine gleichmäßige Beschichtung jedes einzelnen Korns erforderlich ist.

Eine hohe Verdünnung des Behandlungsmittels mit einem organischen Lösungsmittel würde sich positiv hinsichtlich Verteilung des Behandlungsmittels auf die Oberfläche des pulverförmigen Polymeren auswirken, führt aber bei Flüssigkeitsmengen von mehr als 1 Gew.-% zu einem nassen Polymerpulver, das die Fördersysteme bei kontinuierlichen Prozessen verstopft.

Durch eine Erhöhung der Behandlungsmittelmenge auf mehr als 1 Gew.-% wird zwar die Verteilung verbessert, aber ein feuchtes, klebriges Pulver erhalten. Wird dem Behandlungsmittel vor dem Mischprozeß mehr Wasser als zur Herstellung einer ca. 50%igen Lösung benötigt wird zugesetzt, um die Diffusion des Behandlungsmittels in das Polymerkorn zu beschleunigen, so verbackt das pulverförmige Polymere. Durch das in EP 04 50 923 A2 beschriebene kontinuierliche Mischverfahren von wasserquellbaren Polymeren mit Lösungen eines Behandlungsmittels in einem mit speziellen Kunststoffen ausgekleideten Mischer wird zwar das Anhaften des feuchten Polymerpulvers an der Mischerwandung verhindert und die Mischarbeit verringert, aber das Verhalten des Mischproduktes nicht verbessert.

Nach dem Verfahren der EP-PS 0083022 wird die Nachbehandlung wasserhaltiger, gelförmiger Polymerpartikel in organischen Lösungsmitteln durchgeführt. Nach mechanischer Abtrennung des Polymeren erfolgt die Trocknung, wobei zwangsläufig ein Kondensat aus Wasser und organischem Lösungsmittel wie Alkohol, Kohlenwasserstoff, Chlorkohlenwasserstoff oder Keton anfällt, das anschließend so aufgearbeitet werden muß, daß Lösungsmittelanteile weder über die Abluft noch über das Abwasser in die Umwelt gelangen.

Zusammengefaßt ergeben sich für die Beschichtung des pulverförmigen, wasserquellbaren Polymeren mit einer Substanz, die in der Oberflächenschicht des einzelnen Partikels zur Reaktion gebracht werden soll, folgende Bedingungen:

- Die Menge an Behandlungsmittel muß ausreichen, um eine gleichmäßige Beschichtung des Polymerpulvers zu erreichen.

- Die Menge an Wasser, die als Verteilungshilfsmittel und als Träger für das Behandlungsmittel in die Oberflächenschicht des Polymeren dient, ist begrenzt, da es sonst zu irreversiblen Verbackungen der Polymerpartikel kommt.

- Die Gesamtmenge aus Behandlungsmittel, Wasser und gegebenenfalls organischem Lösungsmittel ist begrenzt, da andernfalls nasse, nicht fließfähige Mischungen entstehen.

Betrachtet man den Mischprozeß von wasserquellbaren Polymeren und Behandlungsmittel getrennt vom Gesamtverfahren, so scheint die Verwendung organischer Lösungsmittel zusammen mit dem Behandlungsmittel am sinnvollsten. Die Verteilung des Behandlungsmittels und begrenzter Mengen Wasser auf dem Polymerpulver ist sicher zu erreichen. Auch die Verwendung größerer Behandlungsmittelmengen gewährleistet eine gute Verteilung auf dem Polymeren - auch in Gegenwart von Wasser-, wenn das Behandlungsmittel die Rolle des organischen Lösungsmittels mit übernehmen kann, d. h. ein Verbacken des pulverförmigen Polymeren verhindert. Bei Einsatz zu großer Mengen an Behandlungsmittel kann es aber laut EP 04 50 923 A2 zu einem starken Abfall der Quellkapazität des Polymeren kommen.

Auch bei optimaler Gestaltung des Mischverfahrens von pulverförmigen, wasserquellbaren Polymeren und gegebenenfalls Verdünnungsmitteln muß die Auswirkung auf die nachfolgende Reaktion bei erhöhter Temperatur berücksichtigt werden. Werden die verbesserten Eigenschaften superabsorbierender Polymeren durch nachträgliche Veresterung und/oder Amidierung der Carboxylgruppen des Polymeren erreicht, so sind bei sinnvollen Reaktionszeiten Reaktionstemperaturen von > 150°C erforderlich. Bei diesen Temperaturen werden neben Wasser, das mit 8 - 15 Gew.-% im Ausgangspolymeren enthalten ist, und Lösungsmitteln, auch erhebliche Mengen an Behandlungsmitteln verdampft, die aus dem Reaktor (Trockner) entfernt werden müssen, um eine Kondensation im Reaktor zu verhindern. Der gezielte Transport der Brüden erfolgt mit gegebenenfalls vorgewärmtem Spülgas, da kondensierender Wasserdampf zu Verbackungen und kondensierendes Behandlungsmittel zu Verbackungen und Verfärbungen des pulvertörmigen Polymeren führen würden.

Wasserdampf, verdampfendes Behandlungsmittel, Oxidationsprodukte, Restmonomeren sowie sonstige flüchtige Umwandlungsprodukte und organische Lösungsmittel können aus dem Abgas nur schwierig entfernt werden, d. h. sie gelangen zwangsläufig in die Luft oder in das Abwasser.

Aufgabe der Erfindung ist daher die Bereitstellung von Superabsorbern, die die Eigenschaftskombination von hoher Retentionskapazität, hohe Gelstärker und hohem Aufnahmevermögen unter Druck besitzen und die ohne Verwendung eines organischen Lösungsmittels und mit nur einer geringen Menge Behandlungsmittel für die Nachbehandlung des pulvertörmigen Polymeren hergestellt werden können.

Diese Aufgabe wird gelöst durch die kennzeichnenden Merkmale des Anspruchs 1. Überraschenderweise hat sich gezeigt, daß durch die Verwendung von Phosphorsäure als Verdünnungsmittel für das Mittel, mit dem die Oberfläche des Absorberharzes behandelt wird, Superabsorber mit der gewünschten Eigenschaftskombination bei deutlicher Reduzierung der Mengen an Behandlungsmittel erhalten werden können.

Die Phosphorsäure wird in einer Menge von höchstens 10 Teilen (im nachfolgenden sind alle Teile Gewichtsteile) pro 100 Teile Polymere und in einer Konzentration von mindestens 10 Gew.-% eingesetzt. Bereits mit 0,1 Gew.-% $H_3PO_4$, bezogen auf Polymerpulver, werden erfindungsgemäß verbesserte Superabsorber erhalten.

Als Behandlungsmittel werden erfindungsgemäß eingesetzt:

a) 0,05-0,3 TL einer Verbindung, die mit mindestens zwei Carboxylgruppen des pulverfömigen Polymeren reagieren kann und keine alkalisalzbildende Gruppe enthält, bevorzugt Polyole wie Ethylenglykol, Propandiol, Polyethylenglykol, Glycerin, und Alkylencarbonate wie Ethylencarbonat, und/oder

b) 0,05 - 1 TL einer Verbindung, die mit mindestens zwei Carboxylgruppen des pulverförmigen Polymeren reagieren kann und zusätzlich eine saure, alkalisalzbildende Gruppe im Molekül enthält, z. B. Polyhydroxycarbonsäuren wie Dimethylolpropionsäure (= 2,2-Bis(hydroxymethyl)propionsäure).

Die Behandlungsmittel nach b) haben den Vorteil, daß ihre Flüchtigkeit bei der Reaktion mit den Carboxylgruppen des pulverförmigen Polymeren durch Salzbildung in der Oberflächenschicht des Polymeren eingeschränkt wird.

Das wasserabsorbierende Polymerisat, das zur Beschichtung verwendet werden kann, wird erhalten durch Polymerisation von 55 - 99,9 Gew.-% Monomeren mit Säuregruppen, z. B. Acrylsäure, Methacrylsäure, 2-Acrylamido-2-methylpropansulfonsäure oder Mischungen dieser Monomeren; die Säuregruppen liegen mindestens zu 25 Mol-% neutralisiert vor, so beispielsweise als Natrium-, Kalium- oder Ammoniumsalze. Vorzugsweise liegt der Neutralisationsgrad bei mindestens 50 Mol-%. Besonders bevorzugt ist ein Polymerisat, das aus vernetzter Acrylsäure oder Methacrylsäure gebildet ist, die zu 50 - 80 Mol-% neutralisiert ist.

Als weitere Monomeren können für die Herstellung der wasserabsorbierenden Polymerisate 0 bis 40 Gew.-% Acrylamid, Methacrylamid, Hydroxyethylacrylat, Dimethylaminoalkyl(meth)acrylat, Dimethylaminopropylacrylamid oder Acrylamidopropyltrimethylammoniumchlorid verwendet werden. Höhere Anteile als 40% dieser Monomeren verschlechtern die Quellfähigkeit der Polymerisate.

Als Vernetzer können alle Verbindungen verwendet werden, die mindestens zwei ethylenisch ungesättigte Doppelbindungen oder eine ethylenisch ungesättigte Doppelbindung und eine gegenüber Säuregruppen reaktive funktionelle Gruppe oder mehrere gegenüber Säuregruppen reaktive funktionelle Gruppen tragen. Als Beispiele seien genannt: Acrylate und Methacrylate von Polyolen wie Butandioldiacrylat, Hexandioldimethacrylat, Polyglykoldiacrylat, Trimethylolpropantriacrylat oder Allylacrylat, Diallylacrylamid, Triallylamin, Diallylether, Methylenbisacrylamid oder N-Methylolacrylamid.

Als wasserlösliche Polymeren können im wasserabsorbierenden Polymerisat 0 - 30 Gew.-% teil- oder vollverseifter Polyvinylalkohol, Polyvinylpyrrolodon, Stärke oder Stärkederivate, Polyglykole oder Polyacrylsäuren enthalten sein. Das Molekulargewicht dieser Polymeren ist unkritisch, solange sie wasserlöslich sind. Bevorzugte wasserlösliche Polymeren sind Stärke, Polyvinylalkohol oder Gemische dieser Polymeren. Der bevorzugte Gehalt an solchen wasserlöslichen Polymeren im wasserabsorbierenden Polymerisat liegt bei 1 - 5 Gew.-%, insbesondere wenn Stärke und/oder Polyvinylalkohol als lösliche Polymeren vorhanden sind. Die wasserlöslichen Polymeren können als Pfropfpolymeren mit den säuregruppenenthaltenden Polymeren vorliegen.

Bevorzugt werden neben Polymerisaten, die durch vernetzende Polymerisation teilneutralisierter Acrylsäure erhalten worden sind, solche verwendet, die zusätzlich Anteile von pfropfpolymerisierter Stärke oder von Polyvinylalkohol enthalten.

Hinsichtlich der Teilchenform des eingesetzten Absorber-Polymerisates gibt es keine speziellen Einschränkungen. Das Polymere kann in Form von Kügelchen vorliegen, die durch inverse Suspensionspolymerisation erhalten wurden, oder in Form von unregelmäßig geformten Teilchen, die durch Trocknung und Pulverisierung der Gelmasse aus der Lösungspolymerisation stammen. Die Teilchengröße liegt normalerweise zwischen 20 und 2.000 μm, bevorzugt zwischen 50 und 850 μm.

Die sich an die Beschichtung anschließende thermische Behandlung wird bei 150 - 250 °C durchgeführt, bevorzugt bei 170 - 200 °C. Sie ist abhängig von der Verweilzeit und der Art des Behandlungsmittels. Bei 150 °C muß die thermische Behandlung über mehrere Stunden durchgeführt werden, während bei 250 °C wenige Minuten - z. B. 0,5 - 5 Minuten - ausreichen, um die gewünschten Eigenschaften zu erzielen. Die thermische Behandlung kann in üblichen Trocknern oder Öfen durchgeführt werden; beispielsweise seien genannt: Drehrohröfen, Paddeltrockner, Tellertrockner oder Infrarottrockner.

Die erfindungsgemäßen Polymeren weisen nahe der Oberfläche eine erhöhte Vernetzung und einen verringerten Neutralisationsgrad auf.

Die Polymeren gemäß der Erfindung können in großtechnischer Weise nach kontinuierlichen und diskontinuierlichen Verfahren hergestellt werden. Die erfindungsgemäßen Superabsorber können für breite Anwendungsgebiete eingesetzt werden. Wenn sie z. B. in Damenbinden und Windeln oder zur Wundabdeckung verwendet werden, besitzen sie die Eigenschaft, große Mengen an Menstruationsblut, Urin oder anderen Körperflüssigkeiten schnell zu absorbieren.

Die Absorptionsfähigkeit und -geschwindigkeit unter gleichzeitig einwirkender Druckbelastung ist gegenüber den Ausgangsprodukten stark verbessert. Da die erfindungsgemäßen Superabsorber die absorbierten Flüssigkeiten auch unter Druck zurückhalten, sind sie besonders anwendungsfreundlich. Sie sind bevorzugt geeignet, in höheren Konzentrationen - bezogen auf hydrophiles Fasermaterial wie Fluff- als dies bisher möglich war, eingesetzt zu werden und zeigen ausgezeichnete Absorptionseigenschaften in Konstruktionen, die 98 - 20 Gew.-% hydrophile Fasern und 2 - 80 Gew.-% des Absorberharzes enthalten.

Die erfindungsgemäßen nachbehandelten Polymeren werden in Absorberartikeln für die verschiedensten Anwendungszwecke eingesetzt, z.B. durch Mischen mit Papier, Fluff oder synthetischen Fasern oder Verteilen des Mittels zwischen Substraten aus Papier, Fluff oder nichtgewebten Textilien oder durch Verformung in Trägermaterialien zu einer Bahn.

Die nach dem beschriebenen Verfahren erhaltenen Superabsorber weisen überraschenderweise eine bedeutende Verbesserung der Aufnahme von Flüssigkeit unter Druck hinsichtlich Geschwindigkeit und Gesamtkapazität bei gleichzeitig hoher Gelstärke und hohen Retentionen auf, wobei insbesondere eine sehr hohe Anfangsgeschwindigkeit der Flüssigkeitsaufhahme unter Druck erreicht wird, so daß 80 % der Gesamtkapazität bereits nach 15 Minuten erreicht werden. Die Aufnahme unter Druck, die in DE PS 40 20 780 und EP A 03 39 461 als Absorption under load (AUL) angegeben wird, zeigt eine starke Abhängigkeit von der Höhe der ausgeübten Belastung. Die dort beschriebenen Polymeren haben bei einer Belastung von 1.930,5 Pa (= 0,28 psi = 19.600 dyn/$cm^2$) eine Aufnahmekapazität für 0,9%ige Natriumchloridlösung von 26 bis 34 g/g. Nach EP A 0 339 461 beträgt die Aufnahmekapazität der beschriebenen Polymeren unter einer Belastung von 3.861,1 Pa (= 0,56 psi) maximal 13 g/g und bei einer Belastung mit. 5.860,5 Pa (= 0,85 psi) 8 g/g, d. h. daß die nachbehandelten Polymeren bei einer Belastung von 5.860,5 Pa (= 0,85 psi) nur noch die Menge an Flüssigkeit aufnehmen, die laut DE PS 40 20 780 auch ein nicht nachbehandeltes, wasserquellbares Polymere bei erhöhter Belastung aufnimmt.

Die erfindungsgemäßen Polymeren zeigen bei Belastung mit 40 g/$cm^2$ eine Aufnahmekapazität für 0,9%ige Kochsalzlösung von mindestens 15 g/g, bevorzugt mehr als 18 g/g. Bei einer Belastung mit 60 g/$cm^2$ beträgt die aufgenommene Flüssigkeitsmenge mehr als 12 g/g, bevorzugt mehr als 15 g/g. Dies ist überraschend, da nach DE PS 40 20 780 zur Erhöhung des AUL-Wertes (20 g/$cm^2$) eine Erhöhung der Menge an Nachbehandlungsmittel von 0,5 auf 1,5 Gew.-% erforderlich ist. Diese Maßnahme führt aber, insbesondere wenn noch Wasser zum Lösen des Alkylencarbonats mitverwendet wird, zu einer nassen, pneumatisch nicht mehr förderbaren Mischung und zu hohen Emissionen bei der sich anschließenden thermischen Behandlung.

Die Hersteller textiler Konstruktionen, die der Aufnahme von Körperflüssigkeiten dienen, sind bestrebt, den großvolumigen Faseranteil zu reduzieren und den Anteil an Superabsorber zu erhöhen. Die textile Konstruktion muß aber weiterhin in der Lage sein, die nach der Flüssigkeitsaufnahme gequollenen Teilchen in der textilen Umhüllung bei Belastung zurückzuhalten. Da eine textile Konstruktion zur Aufnahme von Körperflüssigkeit ein hochporöses Gebilde ist, durch dessen Poren ein weiches, gequollenes Gel bei Druckbelastung austreten kann (leakage), ist es eine Aufgabe, wasserquellbare Polymeren, die eine hohe Druckbelastbarkeit zeigen, bereitzustellen.

Die erfindungsgemäßen Polymeren zeigen nicht nur eine erhöhte Absorption für 0,9%ige Natriumchloridlösung unter Druckbelastung, sondern auch eine hohe Aufnahmekapazität für Blut sowie eine schnellere Verteilung des Blutes in einer textilen Konstruktion unter Druckbelastung. Die Polymeren eignen sich daher besonders als Absorbierungsmittel in Damenbinden, da sie die Eigenschaft besitzen, Körperflüssigkeiten wie Blut unter Gewichtsbelastung schnell aufzunehmen. Die Absorptionsgeschwindigkeit für Blut unter gleichzeitig einwirkender Druckbelastung ist viel höher als bei bekannten Produkten.

In einem die Praxis simulierenden Test zur Bestimmung der Saugfähigkeit von Polymeren unter Druck läßt sich zeigen, daß Superabsorber - auch die in der EP A 03 39 461 beschriebenen Polymeren -, die unter einer Belastung von 20 g/cm$^2$ eine hohe Saugkraft zeigen, bei Belastungen von 60 g/cm$^2$ in ihrer Quellfähigkeit deutlich abnehmen. Der Test zeigt weiter, daß wasserquellbare Polymeren, die die gleiche Retention und die gleiche Aufnahmefähigkeit unter einem Druck von 20 g/cm$^2$ haben, sich in ihrer Saugkraft bei erhöhter Belastung unterscheiden können.

Die erfindungsgemäßen Polymeren sind in der Lage, bei einer Belastung von 20 g/cm$^2$ nahezu die gleiche Flüssigkeitsmenge einer textilen Konstruktion zu entziehen wie im unbelasteten Zustand. Das bedeutet, daß z. B. die Fluffschicht einer durch den kindlichen Körper belasteten Windel sicherer und schneller trocken wird und dadurch Feuchtigkeit von der Haut ferngehalten werden kann.

Die dynamische Druckentwicklung, die wasserquellbare Polymeren beim Quellvorgang zeigen, wird als Quelldruck bezeichnet. Beim Quellen steigt dieser Druck an, bis die elektrostatischen Kräfte im Polymeren mit den äußeren mechanischen Kräften im Gleichgewicht stehen.

Die erfindungsgemäßen Polymeren haben einen bis auf das Vierfache erhöhten Quelldruck gegenüber handelsüblichen, bekannten Superabsorbern. Bevorzugt werden wasserquellbare Polymeren mit einem Quelldruck von über 400 g und besonders bevorzugt werden quellbare Polymeren mit einem Quelldruck von mehr als 600 g bei einer Quellfläche von 4,91 cm$^2$.

Die erfindungsgemäßen Polymeren werden wie folgt getestet:

## Testmethoden

Zur Charakterisierung der wasserabsorbierenden Polymerisate wurden Retention (TB) und Aufnahme unter Druck (AUL) für 0,9%ige NaCl-Lösung gemessen sowie die Aufnahmekapazität und die Absorptionsgeschwindigkeit unter Druck für defibriniertes Schafsblut bestimmt.

a) Die Retention wird nach der Teebeutelmethode und als Mittelwert aus drei Messungen angegeben. Etwa 200 mg Polymerisat werden in einen Teebeutel eingeschweißt und für 20 Minuten in 0,9%ige NaCl-Lösung getaucht. Anschließend wird der Teebeutel in einer Schleuder (23 cm Durchmesser, 1.400 Upm) 5 Minuten geschleudert und gewogen. Einen Teebeutel ohne wasserabsorbierendes Polymerisat läßt man als Blindwert mitlaufen.

$$\text{Retention} = \frac{\text{Auswaage - Blindwert}}{\text{Einwaage}} \; g/g$$

b) Die Aufnahme von 0,9%iger NaCl-Lösung unter Druck (Druckbelastung: 20, 40, 60 g/cm$^2$) wird nach der in der EP 03 39 461, Seite 7, beschriebenen Methode bestimmt:

In einen Zylinder mit Siebboden gibt man die Einwaage an Superabsorber und belastet das Pulver mit einem Stempel, der einen Druck von 20, 40 bzw. 60 g/cm$^2$ ausübt. Der Zylinder wird anschließend auf einen Demand-Absorbency-Tester (DAT) gestellt, wo man den Superabsorber eine Stunde lang 0,9%ige NaCl-Lösung aufnehmen läßt.

c) Zur Bestimmung der Aufnahmekapazität für Blut werden ca. 200 mg Polymere in einen Teebeutel eingeschweißt und für 60 Minuten in defibriniertes Schafsblut getaucht und anschließend ausgewogen. Die Berechnung erfolgt wie unter a).

d) Auf ein 6 cm x 20 cm großes Stück Cellulosegewebe (Gewicht: 48,8 g/m$^2$) wird 1 g Polymere gleichmäßig aufgestreut, mit einem Gewebe gleicher Größe abgedeckt und bei 100 °C mit 400 g/cm$^2$ verpreßt.

Der Teststreifen wird zwischen zwei Glasplatten gelegt, von denen die obere im Zentrum eine Bohrung hat. In die Bohrung eingeklebt ist ein Rohrstück von 5,5 cm Länge und 2,2 cm Innendurchmesser. Die obere Platte wird mit Gewichten beschwert, so daß auf dem Teststreifen eine Belastung von 30 g/cm$^2$ liegt. 5 cm$^3$ defibriniertes Schafsblut von 20 °C werden mit einer Schlauchpumpe in 30 Sekunden in das Rohrstuck dosiert und die Einsikkerzeit bestimmt.

e) Die Bestimmung des Quelldrucks Q erfolgt mit Hilfe des Stevens L. F. R. A. Texture Analyser, C. Stevens & Son Ltd, Laboratory Division, St. Albans AL1 1 Ex Hertfordshire, England.

Der zum Gerät gehörende zylindrische Meßkörper aus Glas hat eine Höhe von 3,5 cm und einen Durchmesser von 2,5 cm. Die Kreisfläche des Zylinders beträgt somit 4,91 cm$^2$.

Es werden 0,500 g Superabsorber der Fraktion 20 - 50 mesh in den dazugehörigen Meßzylinder mit 2,7 cm Durchmesser eingewogen und mit 10 ml 0,9%iger NaCl-Lösung versetzt. Danach wird der Meßzylinder mit Hilfe eines Laborboys so weit hochgefahren, bis der Stand der Unterkante des zylindrischen Meßkörpers von der Oberfläche der sich im Meßzylinder befindlichen Probe 12 mm beträgt. Durch die Ausdehnung des Gels wird der Meßzylinder nach oben gegen eine Zwei-Weg-Kraftmeßzelle gedrückt und am Gerät in Gramm angezeigt.

Die Erfindung wird durch die folgenden Beispiele näher erläutert:

**Beispiele**

**A) Herstellung der Mischung aus Polymeren A und Behandlungsmittel**

Eine durch Lösungspolymerisation gewonnene pulverförmige, mit Trimethylolpropantriacrylat vernetzte Polyacrylsäure, die zu 70 Mol-% neutralisiert als Natriumsalz vorlag, wurde nach dem Mahlen auf 90 bis 850 µm abgesiebt (Polymere A).

TB: 36 g/g; Wassergehalt: 10,4 %.

Das Polymere A wird kontinuierlich mit 1.000 kg/h einem Paddelmischer (750 Upm) zugeführt und mit dem Behandlungsmittel vermischt. Anschließend wird die Mischung von einem Fördersystem übernommen und in einen Vorratsbehälter transportiert. Das Erscheinungsbild und damit das Transport- und Lagerverhalten der Fest-flüssig-Mischung wird beurteilt.

Tabelle 1

| Beispiele | Behandlungsmittel | | | Fest-flüssig-Mischung | |
|---|---|---|---|---|---|
| | % | % | % | *Aussehen* | *Verhalten* |
| Vergleich 1 | 0,5 EC | 0,5 H$_2$O | - | trocken | rieselfähig |
| Vergleich 2 | 1,0 EC | 1,0 H$_2$O | - | naß | verbackt |
| Vergleich 3 | 0,25 EC | 0,25 H$_2$O | - | trocken | rieselfähig |
| Vergleich 4 | 0,5 Gl | 0,5 H$_2$O | - | naß | verbackt |
| Vergleich 5 | 0,25 Gl | 0,25 H$_2$O | 1,0 Et | naß | rieselfähig |
| Beispiel 1 | 0,1 EC | 1,0 H$_3$PO$_4$ | - | trocken | rieselfähig |
| Beispiel 2 | 0,1 Gl | 0,6 H$_3$PO$_4$ | 0,3 H$_2$O | trocken | rieselfähig |
| EC: Ethylencarbonat<br>Gl: Glycerin<br>H$_3$PO$_4$ Phosphorsäure 85%ig<br>Et: Ethanol | | | | | |

Die Vergleiche 1 bis 3 entsprechen der DE-PS 40 20 780, die Vergleiche 4 und 5 der DE-OS 35 23 617.

**B) Erwärmung der Mischungen aus Polymeren A und den Behandlungsmitteln**

90 kg/h der nach A) erhaltenen, rieselfähigen, d. h. handhabbaren Mischungen werden kontinuierlich in einen mit Dampf von 180 °C beheizten Paddeltrockner dosiert. Der Trockner hat ein Arbeitsvolumen von 40 l. Die Spülluftmenge zur Abführung der Brüden beträgt ca. 50 m$^3$/h.

Die Kenndaten der erhaltenen, pulverförmigen Polymeren sowie die Fracht an organischen Substanzen im Abgas - angegeben als organischer Kohlenstoff (TOC) - sind in Tabelle 2 aufgelistet.

Tabelle 2

| Beispiel | Mischung aus Beispiel | TB [g/g] | AUL 20 g/cm$^2$ [g/g] | TOC [g/h] |
|---|---|---|---|---|
| Vergleich 6 | Vergleich 1 | 32 | 30 | 115 |
| Vergleich 7 | Vergleich 3 | 33 | 24 | 44 |
| Vergleich 8 | Vergleich 5 | 32 | 31 | 320*) |
| Beispiel 3 | Beispiel 1 | 32 | 30 | 14 |
| Beispiel 4 | Beispiel 2 | 31 | 30 | 4 |

*) Ein Teil des Ethanols verdunstet beim Mischen und Fördern.

## C) Herstellung der Mischung aus Polymeren B und Behandlungsmittel

Das Polymere, das durch Polymerisation einer 30%igen, wäßrigen Acrylsäure, die zu 65 Mol-% neutralisiert als Natriumsalz vorlag, in Gegenwart von 0,28 Gew.-% Triallylamin und 3,5 Gew.-% Polyvinylalkohol gewonnen wurde, wird im Heißluftstrom bei 160 °C getrocknet, gemahlen und auf 120 bis 850 μm abgesiebt (Polymere B).

TB: 37 g/g; Wassergehalt 10,5 %; LA: 11,8%.

Das Polymere B wird wie das Polymere A kontinuierlich mit 1,2 Gew.-% einer 40 °C warmen Lösung aus 0,2 Teilen Dimethylolpropionsäure und 1 Teil 85%iger Phosphorsäure vermischt und in einem Silo zwischengelagert.

## D) Erwärmung der Mischung nach C)

Die nach C) erhaltene rieselfähige Mischung wird mit einer pneumatischen Förderung einem mit rotierenden, mit Dampf von 184 °C beheizten, diskusförmigen Mischelementen ausgerüsteten Trockner zugeführt und anschließend im Wirbelbett abgekühlt. Die Produktdaten und TOC-Werte sind in Tabelle 3 aufgelistet.

Tabelle 3

| Beispiel | Durchsatz [kg/h] | TB [g/g] | AUL [g/g] bei | | | TOC | LA |
|---|---|---|---|---|---|---|---|
| | | | 20 g/cm$^2$ | 40 g/cm$^2$ | 60 g/cm$^2$ | [g/h] | [%]*) |
| 5 | 90 | 32 | 31 | 18 | 14 | 4,5 | 6,7 |
| 6 | 80 | 30 | 30 | 20 | 18 | 5,0 | - |
| 7 | 70 | 28 | 28 | 26 | 23,5 | - | 4,2 |

*) LA: Lösliche Anteile, bestimmt nach EP A 02 05 674.

Tabelle 4

| Bestimmung der Aufnahmekapazität und -geschwindigkeit für Blut | | | |
|---|---|---|---|
| Beispiel | Polymere | Adsorption [g/g] | Adsorptionsgeschwindigkeit bei 30 g/cm$^2$ Belastung [min] |
| 8 | aus Beispiel 3 | 37,5 | 4,5 |
| Vergleich 9 | FAVOR SAB*) FAM | 44 | > 30 |

*) Hersteller: Chemische Fabrik Stockhausen, Krefeld

# EP 0 676 968 B1

## Bestimmung der Saugfähigkeit der Polymeren aus einer Matrix

Ein rundes Fluff-Pad von 6 cm Durchmesser und 2 g Gewicht wird liegend in einer Petrischale mit verschiedenen Mengen 0,9%ige NaCl-Lösung geträrkt. In einen Plexiglaszylinder von 25,8 mm Innendurchmesser, der am Boden ein Siebgewebe hat (Maschenweite 36 μm), werden 0,20 g Polymere eingewogen und mit einem 106 g schweren Stempel von 25 mm Durchmesser belastet. Die Zylindergruppe (Zylinder, Polymere und Stempel) wird gewogen (A) und in die Mitte des befeuchteten Pads gestellt. Nach einer Stunde wird die Zylindergruppe zurückgewogen (B).

$$\text{Saugfähigkeit} = \frac{B - A}{0,20} \text{ g/g}$$

### Tabelle 5

| | Beispiel 9 | Vergleich 10 |
|---|---|---|
| | Polymere nach Beispiel 6<br>TB [g/g]: 30<br>AUL 20 g/cm² [g/g]: 30<br>AUL 60 g/cm² [g/g]: 18 | Polymere nach Vergleich 1<br>TB [g/g]: 32<br>AUL 20 g/cm² [g/g]: 30<br>AUL 60 g/cm² [g/g]: 10 |
| Kochsalzlösung im Pad [g] | Vom Polymeren aufgesaugte Menge NaCl-Lösung [g/g] | |
| 7,5 | 14,0 | 10,0 |
| 15,0 | 20,5 | 13,1 |
| 22,5 | 25,0 | 17,6 |
| 30,0 | 28,9 | 20,3 |

Tabelle 6

| Quelldruckbestimmung | | | | | |
|---|---|---|---|---|---|
| Zeit [min] | 2 | 3 | 5 | 10 | 15 |
| Polymeres aus Beispiel 7 | 200 | 410 | 520 | 820 | 825 |
| FAVOR SAB 800 [1] | 195 | 240 | 250 | 260 | 260 |

[1] Hersteller: Chem. Fab. Stockhausen GmbH, Krefeld

## Patentansprüche

1. Pulverförmiges, vernetztes, wäßrige oder seröse Flüssigkeiten sowie Blut absorbierendes Polymerisat, gebildet aus

   a) 55 - 99,9 Gew.-% polymerisierten ungesättigten, polymerisierbaren, säuregruppenenthaltenden Monomeren, die mindestens zu 25 Mol-% neutralisiert sind,

b) 0 - 40 Gew.-% polymerisierten ungesättigten, mit a) copolymerisierbaren Monomeren,

c) 0,1 - 5,0 Gew.-% eines Vernetzungsmittels,

d) 0 - 30 Gew.-% eines wasserlöslichen Polymeren,
wobei die Gewichtsmengen a) bis d) auf wasserfreie Polymeren bezogen sind, dadurch gekennzeichnet, daß 100 TL der teilchenförmigen Polymeren mit einer wässrigen Lösung aus höchstens 10 TL einer mindestens 10%igen Phosphorsäure und

    a) 0,05 - 0,3 TL einer Verbindung, die mit mindestens zwei Carboxylgruppen reagieren kann und keine alkalisalzbildende Gruppe im Molekül enthält, und/oder

    b) 0,05 - 1 TL einer Verbindung, die mit mindestens zwei Carboxylgruppen reagieren kann und eine alkalisalzbildende Gruppe im Molekül enthält,

vermischt und auf 150 - 250 °C erhitzt worden sind.

**2.** Polymerisat nach Anspruch 1, dadurch gekennzeichnet, daß es

    a) eine Retention von 27 bis 34 g an 0,9%iger Natriumchloridlösung pro Gramm Polymerisat,

    b) eine Aufnahme für 0,9%ige Kochsalzlösung unter einem Druck von 40 g/cm$^2$ von mehr als 15 g, bevorzugt mehr als 18 g pro Gramm Polymerisat,

    c) eine Aufnahme für 0,9%ige Kochsalzlösung unter einem Druck von 60 g/cm$^2$ von mehr als 12 g, bevorzugt mehr als 15 g pro Gramm Polymerisat

    d) einen Quelldruck von mehr als 400 g, bevorzugt mehr als 600 g,
aufweist.

**3.** Polymerisat nach einem der Ansprüche 1 bis 2, dadurch gekennzeichnet, daß es aus Acrylsäure, Methacrylsäure und/oder 2-Acrylamido-2-methylpropansulfonsäure als säuregruppenhaltiges Monomere gebildet ist.

**4.** Polymerisat nach einem der Anprüche 1 bis 3, dadurch gekennzeichnet, daß die säuregruppenenthaltenden Monomeren zu mindestens 50 Mol-% neutralisiert sind.

**5.** Polymerisat nach einem der Ansprüche 1 bis 4, dadurch gekennzeichnet, daß es als einziges säuregruppenenthaltendes Monomere aus Acrylsäure, die zu 50 - 80 Mol-% neutralisiert ist, gebildet ist.

**6.** Polymerisat nach einem der Ansprüche 1 bis 5, dadurch gekennzeichnet, daß wasserlösliche Polymeren in Konzentrationen von 1 bis 5 Gew.-% eingesetzt sind.

**7.** Polymerisat nach einem der Ansprüche 1 bis 6, dadurch gekennzeichnet, daß Stärke und/oder Polyvinylalkohol als wasserlösliche Polymeren eingesetzt sind.

**8.** Verfahren zur Herstellung eines pulverförmigen, vernetzten, wäßrige und seröse Flüssigkeiten sowie Blut absorbierenden Polyinerisates, gebildet aus

    a) 55 - 99,9 Gew.-% polymerisierten ungesättigten, polymerisierbaren, säuregruppenenthaltenden Monomeren, die mindestens zu 25 Mol-% neutralisiert sind,

    b) 0 - 40 Gew.-% polymerisierten ungesättigten, mit a) copolymerisierbaren Monomeren,

    c) 0,1 bis 5,0 Gew.-% eines Vernetzungsmittels,

    d) 0 - 30 Gew.-% eines wasserlöslichen Polymeren,

wobei die Gewichtsmengen a) bis d) aufwasserfreie Polymeren bezogen sind, dadurch gekennzeichnet, daß 100 TL des teilchenförmigen Polymerisats mit einer Lösung aus maximal 10 TL einer mindestens 10%igen Phosphorsäure und

a) 0,05 - 0,3 TL einer Verbindung, die mit mindestens zwei Carboxylgruppen reagieren kann und keine alkalisalzbildende Gruppe im Molekül enthält, und/oder

b) 0,05 - 1 TL einer Verbindung, die mit mindestens zwei Carboxylgruppen reagieren kann und eine alkalisalzbildende Gruppe im Molekül enthält,

vermischt und auf 150 - 250 °C erhitzt werden.

9. Verwendung von Polymerisaten nach Anspruch 1 in textilen Konstruktionen zur Aufnahme von Körperflüssigkeiten unter erhöhter Belastung.

10. Verwendung von Polymerisaten nach Anspruch 9 in Konstruktionen, die aus hydrophilen Fasern und 2 - 80 Gew.-% Polymerisat, bezogen auf das Gesamtgewicht, bestehen.

## Claims

1. A powdered, crosslinked polymer which absorbs aqueous or serous fluids, as well as blood, and has been formed of

a) 55 - 99.9 wt.-% of polymerized unsaturated, polymerizable monomers which contain acid groups and are neutralized to at least 25 mole-%,
b) 0 - 40 wt.-% of polymerized unsaturated monomers copolymerizable with a),
c) 0.1 - 5.0 wt.-% of a crosslinking agent,
d) 0 - 30 wt.-% of a water-soluble polymer,
the weight amounts a) through d) being based on anhydrous polymers,
characterized in that 100 parts of particulate polymers and an aqueous solution of maximally 10 parts of an at least 10% phosphoric acid, and

a) 0.05 - 0.3 parts of a compound which is capable of reacting with at least two carboxyl groups and does not contain any alkali salt-forming groups in its molecule, and/or
b) 0.05 - 1 part of a compound which is capable of reacting with at least two carboxyl groups and contains an alkali salt-forming group in its molecule,

have been mixed and heated to 150 - 250°C.

2. The polymer of claim 1, characterized in that it has

a) a retention of from 27 to 34 g of 0.9% sodium chloride solution per gram of polymer,
b) an absorption for a 0.9% saline solution under a pressure of 40 $g/cm^2$ of more than 15 g, preferably more than 18 g per gram of polymer,
c) an absorption for a 0.9% saline solution under a pressure of 60 $g/cm^2$ of more than 12 g, preferably more than 15 g per gram of polymer,
d) a swelling pressure of more than 400 g, preferably more than 600 g.

3. The polymer according to any one of claims 1 to 2, characterized in that it has been formed of acrylic acid, methacrylic acid and/or 2-acrylamido-2-methylpropanesulfonic acid as monomer containing acid groups.

4. The polymer according to any one of claims 1 to 3, characterized in that the monomers containing acid groups are neutralized to at least 50 mole-%.

5. The polymer according to any one of claims 1 to 4, characterized in that it has been formed of acrylic acid neutralized to 50 - 80 mole-% as the only monomer containing acid groups.

6. The polymer according to any one of claims 1 to 5, characterized in that water-soluble polymers at concentrations of from 1 to 5 wt.-% are used.

7. The polymer according to any one of claims 1 to 6, characterized in that starch and/or poly(vinyl alcohol) are used as water-soluble polymers.

8. A process for producing a powdered, crosslinked polymer which absorbs aqueous and serous fluids, as well as blood, and has been formed of

   a) 55 - 99.9 wt.-% of polymerized unsaturated, polymerizable monomers which contain acid groups and are neutralized to at least 25 mole-%,
   b) 0 - 40 wt.-% of polymerized unsaturated monomers copolymerizable with a),
   c) 0.1 - 5.0 wt.-% of a crosslinking agent,
   d) 0 - 30 wt.-% of a water-soluble polymer,
   the weight amounts a) through d) being based on anhydrous polymers,
   characterized in that 100 parts of particulate polymer and an aqueous solution of maximally 10 parts of an at least 10% phosphoric acid, and

   a) 0,05 - 0.3 parts of a compound which is capable of reacting with at least two carboxyl groups and does not contain any alkali salt-forming groups in its molecule, and/or
   b) 0.05 - 1 part of a compound which is capable of reacting with at least two carboxyl groups and contains an alkali salt-forming group in its molecule,

   are mixed and heated to 150 - 250°C.

9. Use of the polymers according to claim 1 in textile constructions for the absorption of body fluids under elevated load.

10. The use of polymers according to claim 9 in constructions comprised of hydrophilic fibers and 2-80 wt.-% of polymer, relative to the total weight.

**Revendications**

1. Polymère pulvérulent, réticulé, absorbant des liquides aqueux ou séreux ainsi que du sang, formé à base

   a) de 55-99,9 % en poids de monomères insaturés polymérisés, polymérisables, contenant des groupes acides, qui sont neutralisés au moins pour 25 % en moles,
   b) de 0-40 % en poids de monomères insaturés polymérisés, copolymérisables avec a),
   c) de 0,1-5,0 % en poids d'un agent réticulant,
   d) de 0-30 % en poids d'un polymère soluble dans l'eau,
   les proportions en poids a) à d) étant rapportées au polymère anhydre, caractérisé en ce que 100 parties du polymère sous forme de particules ont été mélangées à une solution aqueuse d'au maximum 10 parties d'un acide phosphorique à au moins 10 %, et

   a) à 0,05-0,3 partie d'un composé qui peut réagir avec au moins deux groupes carboxyliques et qui ne contient dans la molécule aucun groupe formateur de sel alcalin, et/ou
   b) à 0,05-1 partie d'un composé, qui peut réagir avec au moins deux groupes carboxyliques et qui contient dans la molécule un groupe formateur de sel alcalin,

   et en ce qu'elles ont été chauffées à 150-250°C.

2. Polymère suivant la revendication 1, caractérisé en ce qu'il présente

   a) une rétention de 27 à 34 g sur une solution de chlorure de sodium à 0,9 % par g de polymère,
   b) une absorption de solution de chlorure de sodium à 0,9 % sous une pression de 40 $g/cm^2$ de plus de 15 g, avantageusement de plus de 18 g, par gramme de polymère,
   c) une absorption de solution de chlorure de sodium à 0,9 % sous une pression de 60 $g/cm^2$ de plus de 12 g, de préférence de plus de 15 g, par gramme de polymère,
   d) une pression de gonflement de plus de 400 g, de préférence de plus de 600 g.

3. Polymère suivant l'une des revendications 1 et 2, caractérisé en ce qu'il est formé à partir d'acide acrylique, d'acide méthacrylique et/ou d'acide 2-acrylamido-2-méthylpropanesulfonique, comme monomères contenant des groupes acides.

4. Polymère suivant l'une des revendications 1 à 3, caractérisé en ce que les monomères contenant des groupes acides sont neutralisés à au moins 50 % en moles.

5. Polymère suivant l'une des revendications 1 à 4, caractérisé en ce qu'il est formé à partir d'acide acrylique qui est neutralisé à 50-80 % en moles, comme seul monomère contenant des groupes acides.

6. Polymère suivant l'une des revendications 1 à 5, caractérisé en ce que des polymères solubles dans l'eau sont mis en oeuvre en des concentrations de 1 à 5 % en poids.

7. Polymère suivant l'une des revendications 1 à 6, caractérisé en ce que de l'amidon et/ou de l'alcool polyvinylique sont mis en oeuvre comme polymères solubles dans l'eau.

8. Procédé de préparation d'un polymère pulvérulent, réticulé, absorbant des liquides aqueux et séreux ainsi que du sang, et formé à base

   a) de 55-99,9 % en poids de monomères insaturés polymérisés, polymérisables, contenant des groupes acides, qui sont neutralisés à au moins 25 % en moles,
   b) de 0-40 % en poids de monomères insaturés polymérisés, copolymérisables avec a),
   c) de 0,1 à 5,0 % en poids d'un agent réticulant,
   d) de 0-30 % en poids d'un polymère soluble dans l'eau,
   les proportions pondérales a) à d) étant rapportées au polymère anhydre,
   caractérisé en ce qu'à 100 parties du polymère en forme de particules on mélange une solution à base d'au maximum 10 parties d'un acide phosphorique à au moins 10 %, et

   a) 0,05-0,3 partie d'un composé, qui peut réagir avec au moins deux groupes carboxyliques et qui ne contient pas de groupes formateurs de sel alcalin dans la molécule, et/ou
   b) 0,05-1 partie d'un composé, qui peut réagir avec au moins deux groupes carboxyliques et qui contient un groupe formateur de sel alcalin dans la molécule,

   et en ce qu'on chauffe le tout à 150-250°C.

9. Utilisation de polymères suivant la revendication 1 dans des produits textiles destinés à l'absorption de liquides corporels, sous charge élevée.

10. Utilisation de polymères suivant la revendication 9 dans des constructions qui sont constituées de fibres hydrophiles et de 2-80 % en poids de polymère, par rapport au poids total.